# EUROPÄISCHE PATENTSCHRIFT

(11) **EP 1 184 029 B1**
(45) Veröffentlichungstag und Bekanntmachung des Hinweises auf die Patenterteilung: **08.08.2007**
(21) Anmeldenummer: 00118953.9
(22) Anmeldetag: 01.09.2000
(51) Int. Cl.: A61Q 11/00, A61K 8/11, A61K 8/73

(54) **Mund- und Zahnpflegemittel**
Oral and dental care composition
Composition de soins bucco-dentaires

(43) Veröffentlichungstag der Anmeldung: 06.03.2002
(73) Patentinhaber: Cognis IP Management GmbH, 40589 Düsseldorf (DE)
(72) Erfinder: de Moragas, Maria, Dr., 08310 Argentona, Barcelona (ES); Amela Conesa, Christina, 08029 Cerdanyola del Vallés, Barcelona (ES); Viladot Petit, Josep-Lluis, Dr., 08018 Barcelona (ES)

(56) Entgegenhaltungen:
- WO-A-99/04764
- US-A- 5 711 938
- DATABASE CAPLUS [Online] LEE ET AL.: "polysaccharide microcapsules for the treatment of periodontitis" retrieved from STN Database accession no. 1996:522033 XP002159886
- LEE S.J. ET AL: 'Polysaccharide Microcapsules for the Treatment of Periodontitis' PROCEED. INTERN. SYMP. CONTROL. REL. BIOACT. MATER. Bd. 23, 1996, CONTROLLED RELEASE SOCIETY, INC., Seiten 373 - 374

## Beschreibung

### Gebiet der Erfindung

Die Erfindung befindet sich auf dem Gebiet der Mund- und Zahnhygiene und betrifft Zubereitungen, die sich durch eine wirksame Menge von mit Wirkstoffen beladenen Chitosanmikrokapseln auszeichnen.

### Stand der Technik

Unter dem Begriff Mund- und Zahnpflegemittel, der im englischen Sprachbereich gerne unter dem Begriff "Oral Care" zusammengefasst wird, versteht der Fachmann sehr unterschiedliche Produkte in ebenso unterschiedlichen Anbietungsformen. Im einfachsten Fall handelt es sich hierbei um Zahnpasten oder Mundwasser, wobei erstere als mehr oder minder viskose Pasten in einer Tube oder einem Spender angeboten werden, während letztere in der Regel als Flüssigkeiten in den Handel gelangen, die entweder in Wasser aufgelöst oder direkt in den Rachenraum gesprüht werden. Zwischen diesen beiden Formen gibt es jedoch zusehends mehr Produkte, die dem "2-in-1-Prinzip" folgen, d.h. Zahnbehandlungsmittel, die zusätzlich auch eine desinfizierende oder desodorierende Wirkung haben und sehr gerne in Form von halbflüssigen Gelen formuliert werden oder Mundwassern, denen man Wirkstoffe zum Vorbeugen gegen Zahnerkrankungen zugibt.

Die für diese Zwecke geeigneten Wirkstoffe, Feuchthaltemittel, Tenside, Schleif- und Poliermittel, Aromakomponenten und dergleichen sind hinlänglich bekannt, die Zahl der einschlägigen Patente zu diesem Thema Legion. Nichtsdestotrotz besteht nach wie vor der Wunsch, Zubereitungen zu formulieren, die bei gleicher Leistung bzw. Schutzwirkung mit geringeren Wirkstoffmengen auskommen, bzw. bei denen man die Inhaltsstoffe durch eine besondere Darbietungsform besonders effektiv zur Anwendung bringen kann.

In diesem Zusammenhang sei auf die WO 00/16735 (Cognis) hingewiesen, aus der Zahnpasten bekannt sind, die Chitosan zusammen mit Monoglyceridsulfaten enthalten. Aus der EP 0758223 A1 (Medicarb) sind ähnliche Zubereitungen bekannt, welche Kombinationen von Chitosan mit negativ geladenen Polysacchariden enthalten. Gegenstand der DE 3343200 A1 (Lion) ist die Verwendung von wasserlöslichen Chitosansalzen zur Herstellung von Mundwassern.

Lee, S. J. et al. (Proceed. Intern. Symp. Control. Rel. Bioact. Mater. 23 (1996), Controlled Release Society Inc., offenbart mit Minocycline. HCl beladene und mit Chitosan beschichtete Mikrokapseln aus Na-Alginat zur Behandlung von Periodontitis.

Die Aufgabe der vorliegenden Erfindung hat somit darin bestanden, neue Zubereitungen für die Mund- und Zahnhygiene zur Verfügung zu stellen, die gegenüber dem Stand der Technik einen verbesserten Schutz gegen Karies, Periodontosis und Plaque bieten und dabei gleichzeitig anti-inflammatorisch wirksam sind.

### Beschreibung der Erfindung

Gegenstand der Erfindung sind Mund- und Zahnpflegemittel, enthaltend mit Wirkstoffen und Gelbildnern ausgewählt aus der Gruppe der Agarosen, Pektine, Xanthane und Proteine beladene Chitosanmikrokapseln, die nach den in Anspruch 1 genannten Verfahren erhältlich sind.

Überraschenderweise wurde gefunden, dass die Zubereitungen infolge der Verkapselung der Wirkstoffe gegenüber vergleichbaren Produkten des Marktes eine verbesserte Kariesinhibierung und Periodontosisprophylaxe sowie eine höhere Antiplaquewirkung aufweisen und dabei gleichzeitig anti-inflammatorisch wirksam sind. Die Erfindung schließt dabei die Erkenntnis ein, dass für die besondere Wirkung die Auswahl von Chitosan als Verkapselungsmittel entscheidend ist.

### Wirkstoffe

Die Auswahl der zu verkapselnden Wirkstoffe ist für die Lehre der Erfindung zwar von Bedeutung, jedoch nicht entscheidend, da es darauf ankommt, für diesen Zweck hinlänglich bekannte Einsatzstoffe in einer neuen, effizienten Anbietungsform zum Einsatz zu bringen, d.h. in einer Matrix aus Chitosan zu verkapseln. Demzufolge kann der Begriff Wirkstoff sehr breit ausgelegt werden und letztlich alle üblichen Hilfs- und Zusatzstoffe umfassen, die für eine Anwendung im Bereich der Mund- und Zahnhygiene vom Fachmann in Betracht gezogen werden, also beispielsweise Wirkstoffe gegen Erkrankungen der Zähne, der Zahnhälse und des Zahnfleischs (Karies, Periodontosis, Plaque), antiinflammatorische Mittel, Aromakomponenten, Farbstoffe, aber gegebenenfalls auch Schleif- und Poliermittel, Feuchthalte- und Bindemittel, Verdicker, Bakterizide und Tenside - freilich nur soweit diese für die orale Anwendung zugelassen sind - wenngleich diese eher unverkapselt eingesetzt werden. Insofern besitzt die folgende Auflistung lediglich einen beispielhaften, nicht aber einen abschließenden Charakter.

### Wirkstoffe für die Zahngesundheit

Als kariesprophylaktische Wirkstoffe enthalten die meisten Mund- und Zahnpflegemittel heute meistens eine oder zwei Fluorverbindungen, teilweise noch in Kombination mit Stoffen, die eine Remineralisierung fördern. Natriummonofluorphosphat und Natriumfluorid sind die am häufigsten eingesetzten Fluorverbindungen, es werden aber auch Aluminiumfluorid, Zinn-(II)-fluorid sowie Aminfluoride, wie z.B. Bis(hydroxyethyl)aminopropyl-N-hydroxyethyl-octyldecylamin-dihydrofluorid oder Cetylaminhydrofluorid eingesetzt. Als remineralisierende Stoffe können u.a. Calciumglycerophosphat, Calciumhydrogenphosphat, cyclisches Natriumtrimetaphosphat oder Alkaliorthophosphate verwendet werden. Zur Pflege des Zahnfleisches werden vor allem folgende Substanzen verkapselt: Allantoin, Azulen, Bisabolol, Carbamid, Panthenol, Extrakte aus Arnike, Myrrhe, Ratanhia, Salbei, Echinacea, Kamille, Aloe, Neem, Calendula, Roßkastanienrinde, Tormentill, Rosmarin, Hamamelis und Malve sowie Ammoniumglycyrrhicinat, Aluminiumlactat, β-Pyridylcarbinoltartrat, Vitamin A, Glycyrrhetinsäure, Eugenol, Meersalz, Prenylaminlactat und Benzylnicotinat. Folgende Stoffe werden verkapselt und können die Neubildung von weichen und harten Zahnbelägen reduzieren : Zinkaspartat, Zinkcitrat, Domiphenbromoid, Cetylpyridiniumchlorid, Hexetidin, Trichlosan, Chlorhexidindigluconat, Natriumsulforicinoleat, Azacycloheptandiphosphonat, Zinkchlorid, Alkalidiphosphate sowie bestimmte Enzyme. Zubereitungen gegen empfindliche Zähne können schließlich in verkapselter Form Strontiumchlorid, Kaliumnitrat, Kaliumchlorid, Natriumcitrat, Citronensäure oder Hydroxylapatit enthalten.

### Aromakomponenten

Als Aromakomponenten kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage. Als Süßstoffe können Saccharin-Natrium, gegebenenfalls in Kombination mit Natriumcyclamat, Acesulfam® K oder Aspartame® eingesetzt werden.

### Farbstoffe

Die für die Verkapselung in Frage kommenden Farbstoffe können sowohl wasserlöslich als auch wasserunlöslich sein. Während zur Herstellung von mehrfarbigen Zubereitungen üblicherweise wasserunlösliche Farblacke oder Pigmente verwendet werden müssen, um eine Vermischung zu verhindern, können im Sinne der Erfindung zu diesem Zweck sehr wohl auch wasserlösliche Farbstoffe verwendet werden, da diese ja durch die Chitosankapsel geschützt sind. Die am häufigsten vewendeten Farbstoffe sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein.

### Chitosanmikrokapseln

Unter dem Begriff "Mikrokapsel" werden vom Fachmann sphärische Aggregate mit einem Durchmesser im Bereich von etwa 0,1 bis etwa 5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden flüssige Wirkstoffe in einer Matrix aufgenommen ("microsponge"), die als Mikropartikel zusätzlich mit filmbildenden Polymeren umhüllt sein können. Die mikroskopisch kleinen Kapseln, auch Nanokapseln genannt, lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccaride, wie Stärke oder Dextran, Polypeptide, Proteinghydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide).

In diesem Zusammenhang sei auch auf die deutsche Patentanmeldung DE 19712978 A1 (Henkel) hingewiesen, aus der Chitosanmikrosphären bekannt sind, die man erhält, indem man Chitosane oder Chitosanderivate mit Ölkörpern vermischt und diese Mischungen in alkalisch eingestellte Tensidlösungen einbringt. Aus der deutschen Patentanmeldung DE 19756452 A1 (Henkel) ist ferner auch die Verwendung von Chitosan als Verkapselungsmaterial für Tocopherol bekannt. Chitosanmikrokapseln und Verfahren zu ihrer Herstellung sind Gegenstand früherer Patenanmeldungen der Pantentanmelderin. Man unterscheidet dabei im wesentlichen die beiden folgenden Verfahren:
(1) Mikrokapseln mit mittleren Durchmessern im Bereich von 0,1 bis 5 mm, bestehend aus einer Hüllmembran und einer mindestens einen Wirkstoff enthaltenden Matrix, erhältlich, indem man
   (a) aus Gelbildnern, Chitosanen und Wirkstoffen eine Matrix zubereitet,
   (b) gegebenenfalls die Matrix in einer Ölphase dispergiert,
   (c) die dispergierte Matrix mit wäßrigen Lösungen anionischer Polymere behandelt und gegebenenfalls dabei die Ölphase entfernt.
(2) Mikrokapseln mit mittleren Durchmessern im Bereich von 0,1 bis 5 mm, bestehend aus einer Hüllmembran und einer mindestens einen Wirkstoff enthaltenden Matrix, erhältlich, indem man
   (a) aus Gelbildnern, anionischen Polymeren und Wirkstoffen eine Matrix zubereitet,
   (b) gegebenenfalls die Matrix in einer Ölphase dispergiert,
   (c) die dispergierte Matrix mit wäßrigen Chitosanlösungen behandelt und gegebenenfalls dabei die Ölphase entfernt.

### Gelbildner

Als Gelbildner werden solche Stoffe in Betracht gezogen, welche die Eigenschaft zeigen in wäßriger Lösung bei Temperaturen oberhalb von 40 °C Gele zu bilden, und welche ausgewählt sind aus der Gruppe der Agarosen, Pektine, Xanthane und Proteine. Als thermogelierende Heteropolysaccharide kommen Agarosen in Frage, welche in Form des aus Rotalgen zu gewinnenden Agar-Agar auch zusammen mit bis zu 30 Gew.-% nichtgelbildenden Agaropektinen vorliegen können. Hauptbestandteil der Agarosen sind lineare Polysaccharide aus D-Galaktose und 3,6-Anhydro-L-galaktose, die alternierend β-1,3- und β-1,4-glykosidisch verknüpft sind. Die Heteropolysaccharide besitzen vorzugsweise ein Molekulargewicht im Bereich von 110.000 bis 160.000 und sind sowohl farb- als auch geschmacklos. Als Alternativen kommen Pektine, Xanthane (auch Xanthan Gum) sowie deren Mischungen in Frage. Es sind weiterhin solche Typen bevorzugt, die noch in 1-Gew.-%iger wäßriger Lösung Gele bilden, die nicht unterhalb von 80 °C schmelzen und sich bereits oberhalb von 40 °C wieder verfestigen. Aus der Gruppe der thermogelierenden Proteine seien exemplarisch die verschie-denen Gelatine-Typen genannt.

### Chitosane

Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten:

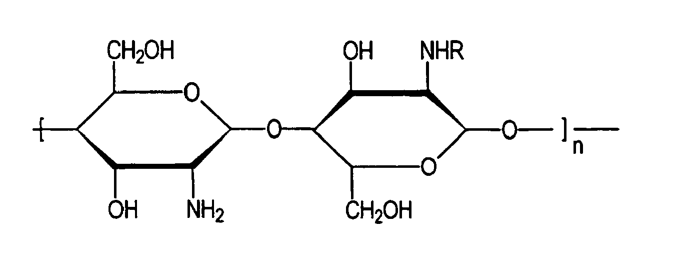

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt (vgl. Ullmann's Encyclopedia of Industrial Chemistry, 5th Ed., Vol. A6, Weinheim, Verlag Chemie, 1986, S. 231-232**).** Übersichten zu diesem Thema sind auch beispielsweise von B. Gesslein et al. in HAPPI 27, 57 (1990**),** O. Skaugrud in Drug Cosm.Ind. 148, 24 (1991**)** und E. Onsoyen et al. in Seifen-Öle-Fette-Wachse 117, 633 (1991**)** erschienen. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren, das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Entsprechende Verfahren sind beispielsweise aus Makromol. Chem. 177, 3589 (1976**)** oder der französischen Patentanmeldung FR 2701266 A bekannt. Vorzugsweise werden solche Typen eingesetzt, wie sie in den deutschen Patentanmeldungen DE 4442987 A1 und DE 19537001 A1 (Henkel) offenbart werden und die ein durchschnittliches Molekulargewicht von 10.000 bis 500.000 bzw. 800.000 bis 1.200.000 Dalton aufweisen und/oder eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% besitzen. Aus Gründen der besseren Wasserlöslichkeit werden die Chitosane in der Regel in Form ihrer Salze, vorzugsweise als Glycolate eingesetzt.

### Anionische Polymere

Die anionische Polymeren haben die Aufgabe, mit den Chitosanen Membranen zu bilden. Je nach Herstellverfahren können sie in der Matrix enthalten sein (dann erfolgt die Membranbildung durch Behandlung mit den Chitosanlösungen) oder als Fällungsmittel für die in der Matrix enthaltenen Chitosane dienen. Als anionische Polymere eignen sich vorzugsweise Salze der Alginsäure. Bei der Alginsäure handelt es sich um ein Gemisch carboxylgruppenhaltiger Polysaccharide mit folgendem idealisierten Monomerbaustein:

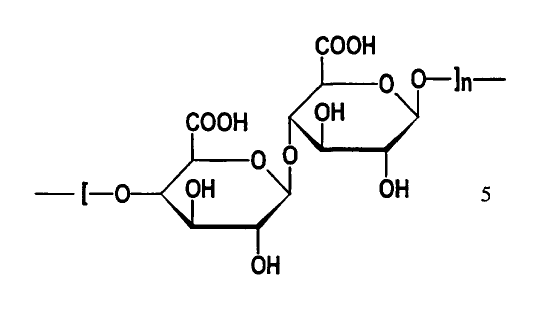

Das durchschnittliche Molekulargewicht der Alginsäuren bzw. der Alginate liegt im Bereich von 150.000 bis 250.000. Dabei sind als Salze der Alginsäure sowohl deren vollständige als auch deren partiellen Neutralisationsprodukte zu verstehen, insbesondere die Alkalisalze und hierunter vorzugsweise das Natriumalginat ("Algin") sowie die Ammonium- und Erdalkalisalze. besonders bevorzugt sind Mischalginate, wie z.B. Natrium/Magnesium- oder Natrium/Calciumalginate. In einer alternativen Ausführungsform der Erfindung kommen für diesen Zweck jedoch auch anionische Chitosanderivate, wie z.B. Carboxylierungs- und vor allem Succinylierungsprodukte in Frage, wie sie beispielsweise in der deutschen Patentschrift DE 3713099 C2 (L'Oréal) sowie der deutschen Patentanmeldung DE 19604180 A1 (Henkel) beschrieben werden.

Zur Herstellung der Chitosanmikrokapseln stellt man beispielsweise eine 1 bis 10, vorzugsweise 2 bis 5 Gew.-%ige wäßrige Lösung des Gelbildners, vorzugsweise des Agar-Agars her und erhitzt diese unter Rückfluß. In der Siedehitze, vorzugsweise bei 80 bis 100°C, wird eine zweite wäßrige Lösung zugegeben, welche das Chitosan in Mengen von 0,1 bis 2, vorzugsweise 0,25 bis 0,5 Gew.-% und den Wirkstoff in Mengen von 0,1 bis 25 und insbesondere 0,25 bis 10 Gew.-% enthält; diese Mischung wird als Matrix bezeichnet. Die Beladung der Mikrokapseln mit Wirkstoffen kann daher ebenfalls 0,1 bis 25 Gew.-% bezogen auf das Kapselgewicht betragen. Falls gewünscht, können zu diesem Zeitpunkt zur Viskositätseinstellung auch wasserunlösliche Bestandteile, beispielsweise anorganische Pigmente zugegeben werden, wobei man diese in der Regel in Form von wäßrigen oder wäßrig/alkoholischen Dispersionen zusetzt. Zur Emulgierung bzw. Dispergierung der Wirkstoffe kann es ferner von Nutzen sein, der Matrix Emulgatoren und/oder Lösungsvermittler hinzuzugeben.

### Emulgatoren

Als Emulgatoren kommen beispielsweise nichtionogene Tenside aus mindestens einer der folgenden Gruppen in Frage:
➢ Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
➢ Alkyl- und/oder Alkenyloligoglykoside mit 8 bis 22 Kohlenstoffatomen im Alk(en)ylrest und deren ethoxylierte Analoga;
➢ Anlagerungsprodukte von 1 bis 15 Mol Ethylenoxid an Ricinusöl und/oder gehärtetes Ricinusöl;
➢ Anlagerungsprodukte von 15 bis 60 Mol Ethylenoxid an Ricinusöl und/oder gehärtetes Ricinusöl;
➢ Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Mischester aus Pentaerythrit, Fettsäuren, Citronensäure und Fettalkohol gemäß DE 1165574 **PS** und/oder Mischester von Fettsäuren mit 6 bis 22 Kohlenstoffatomen, Methylglucose und Polyolen, vorzugsweise Glycerin oder Polyglycerin.
➢ Mono-, Di- und Trialkylphosphate sowie Mono-, Di- und/oder Tri-PEG-alkylphosphate und deren Salze;
➢ Wollwachsalkohole;
➢ Polysiloxan-Polyalkyl-Polyether-Copolymere bzw. entsprechende Derivate;
➢ Polyalkylenglycole sowie
➢ Glycerincarbonat.

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus DE 2024051 **PS** als Rückfettungsmittel für kosmetische Zubereitungen bekannt.

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.
Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische.

Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin.

Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

Als Lösungsvermittler oder Hydrotrope eignen sich beispielsweise Ethanol, Isopropylalkohol, oder Polyole eingesetzt werden. Letztere besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
➢ Glycerin;
➢ Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
➢ technische Oligoglyceringemische mit einem Eigenkondensationsgrad von 1,5 bis 10 wie etwa technische Diglyceringemische mit einem Diglyceringehalt von 40 bis 50 Gew.-%;
➢ Methyolverbindungen, wie insbesondere Trimethylolethan, Trimethylolpropan, Trimethylolbutan, Pentaerythrit und Dipentaerythrit;
➢ Niedrigalkylglucoside, insbesondere solche mit 1 bis 8 Kohlenstoffen im Alkylrest, wie beispielsweise Methyl- und Butylglucosid;
➢ Zuckeralkohole mit 5 bis 12 Kohlenstoffatomen, wie beispielsweise Sorbit oder Mannit,
➢ Zucker mit 5 bis 12 Kohlenstoffatomen, wie beispielsweise Glucose oder Saccharose;
➢ Aminozucker, wie beispielsweise Glucamin;
➢ Dialkoholamine, wie Diethanolamin oder 2-Amino-1,3-propandiol.

Die Konzentration der Emulgatoren kann bezogen auf die Wirkstoffe 1 bis 20 und vorzugsweise 5 bis 10 Gew.-% betragen. Die Menge an Lösungsvermittler richtet sich ausschließlich nach der Wasserlöslichkeit bzw. Wasserdispergierbarkeit der Wirkstoffe.

### Herstellung der Mikrokapseln

Nach der Herstellung der Matrix aus Gelbildner, Chitosan und Wirkstoff wird in einer besonderen Ausführungsform des Verfahrens die Matrix in einer Ölphase unter starker Scherung sehr fein dispergiert, um bei der nachfolgenden Verkapselung möglichst kleine Teilchen herzustellen. Dabei hat es sich als besonders vorteilhaft erwiesen, die Matrix auf Temperaturen im Bereich von 40 bis 60 °C zu erwärmen, während man die Ölphase auf 10 bis 20 °C kühlt. Im dritten Schritt erfolgt dann die eigentliche Verkapselung, d.h. die Ausbildung der Hüllmembran durch Inkontaktbringen des Chitosans in der Matrix mit den anionischen Polymeren. Hierzu empfiehlt es sich, die in der Ölphase dispergierte Matrix bei einer Temperatur im Bereich von 40 bis 100, vorzugsweise 50 bis 60 °C mit einer wäßrigen, etwa 0,1 bis 3 und vorzugsweise 0,25 bis 0,5 Gew.-%ige wäßrigen Lösung des Anionpolymers, vorzugsweise des Alginats zu waschen und dabei gleichzeitig die Ölphase zu entfernen.

In gleicher Weise ist es möglich, im ersten Schritt eine Matrix aus Gelbildner, Anionpolymer und Wirkstoff herzustellen, die Matrix in einer Ölphase zu dispergieren und dann die Kapseln durch Fällung mit einer Chitosanlösung herzustellen. Dazu reicht es aus, in der obigen Herstellvorschrift nur jeweils "Anionpolymer" und "Chitosan" zu vertauschen und die Mengenangaben beizubehalten. In zwei weiteren alternativen Ausführungsformen kann jeweils auf die Dispergierung in einer Ölphase verzichtet werden, dann werden jedoch in der Folge eher größere Kapseln erhalten. Somit stehen zur Herstellung der Chitosanmikrokapseln insgesamt vier Verfahren zur Verfügung. Die dabei resultierenden wäßrigen Zubereitungen weisen in der Regel einen Mikrokapselgehalt im Bereich von 1 bis 10 Gew.-% auf. In manchen Fällen kann es dabei von Vorteil sein, wenn die Lösung der Polymeren weitere Inhaltsstoffe, beispielsweise Emulgatoren oder Konservierungsmittel enthält. Nach Filtration werden Mikrokapseln erhalten, welche im Mittel einen Durchmesser im Bereich von vorzugsweise 1 bis 3 mm aufweisen. Es empfiehlt sich, die Kapseln zu sieben, um eine möglichst gleichmäßige Größenverteilung sicherzustellen. Die so erhaltenen Mikrokapseln können im herstellungsbedingten Rahmen eine beliebige Form aufweisen, sie sind jedoch bevorzugt näherungsweise kugelförmig.

### Gewerbliche Anwendbarkeit

Ein weiterer Gegenstand der Erfindung betrifft daher die Verwendung der mit Wirkstoffen beladenen Chitosanmikrokapseln nach Anspruch 1 zur Herstellung von Mund- und Zahnpflegemitteln, die diese in Mengen von 1 bis 10, vorzugsweise 2 bis 15 und insbesondere 5 bis 10 Gew.-% -bezogen auf die Mittel - enthalten können.

### Hilfs- und Zusatzstoffe

Die Zubereitungen können neben den Chitosanmikrokapseln weitere nicht-verkapselte Hilfs- und Zusatzstoffe enthalten, die im wesentlichen mit den auch für die Verkapselung in Frage kommenden Wirkstoffen und dergleichen identisch sein können. Dabei ist es auch möglich verkapselte und unverkapselte Wirkstoffe nebeneinander einzusetzen. Obschon grundsätzlich auch für eine Verkapselung in Frage kommend, enthalten die erfindungsgemäßen Mittel die folgenden Zusatzstoffe in typischen Mengen jedoch bevorzugt unverkapselt:

### Schleif- und Poliermittel

Unter Schleif- und Poliermitteln sind in der Regel wasserunlösliche, anorganische Stoffe zu verstehen, die vor allem in Zahnpasten in Konzentrationen von typischerweise 15 bis 60 Gew.-% enthalten sind. Aufgabe dieser Stoffe, die auch als Abrasivstoffe oder Putzkörper bezeichnet werden, ist es, die mechanische Reinigungswirkung der Zahnbürste zu unterstützen und den Zeitaufwand für die Reinigung der zugänglichen Zahnoberfläche zu reduzieren, wobei der reinigende Effekt im wesentlichen auf der Entfernung von Plaque beruht. Typische Beispiele für geeignete Schleif- und Poliermittel sind beispielsweise Kreide, Dicalcium-phosphat, unlösliches Natriummetaphosphat, Aluminiumsilicat, Schichtsilicate, Hydrotalcite, Calciumpyrophosphat, feinteilige Polymere (z.B. Polymethylmethacrylat), Kieselsäuren, Aluminiumoxid, Aluminiumoxidtrihydrat, Talkum, Zeolithe, Magnesiumaluminiumsilicat, Calciumsulfat, Magnesiumcarbonat und/oder Magnesiumoxid, wobei die mittlere Teilchengröße üblicherweise unter 15 µm liegt. Darüber hinaus kann auch wasserlösliches Natriumhydrogencarbonat, meistens im Gemisch mit Calciumcarbonat, eingesetzt werden. Das NaHCO₃, das auch als temporärer Putzkörper bezeichnet wird, geht beim Zähneputzen langsam im Speichel in Lösung.

### Feuchthaltemittel

Abgesehen von reinen Zahnpulvern enthalten praktisch alle erfindungsgemäßen Zubereitungen, insbesondere aber alle Mikrokapseln aufweisende Zahnpasten Feuchthaltemittel, die ein Austrocknen verhindern und die Kältestabilität verbessern sollen. Die am hierzu am häufigsten verwendeten Stoffe sind Glycerin, Sorbit, Xylit, 1,2-Propylenglycol sowie die vielfältigen Polyethylenglycole.

### Bindemittel und Verdicker

Binde- und Verdickungsmittel geben insbesondere cremeförmigen Zubereitungen die Struktur und verhindern die Separierung von flüssigen und festen Anteilen. Für diesen zweck eignen sich insbesondere hydrophile Kolloide natürlichen und synthetischen Ursprungs wie Alginate, Carrageenan, Tragant, Natriumcarboxymethylcellulose, Hydroxyethylcellulose, Methylcellulose, Methylhydroxypropylcellulose, Xanthan Gum und Guar Gum. Häufig verwendet werden auch hochdisperses Siliciumdioxid, Magnesiumaluminiumsilicat, Magnesiumlithiumsilicat oder Bentonit.

### Tenside

Der Zusatz von Tensiden ist in der Regel erforderlich, um den Produkten ein hinreichendes Anschäumvermögen zu verleihen. Für diesen Zweck können natürlich nur solche oberflächenaktiven Verbindungen eingesetzt werden, die die physiologisch unbedenklich und im wesentlichen geschmacksneutral sind. Die am häufigsten verwendeten Mittel sind Natriumlaurylsulfat, Natriumlaurylsarcosinat, medizinische Seife, Palmkernfettsäuretaurid, Natriumlaurylsulfoacetat, Kokosfettsäuremonoglyceridsulfat und Betaine.

### Bakterizide und Konservierungsmittel

Bakterizide werden den Mitteln in der Regel zum Schutz vor mikrobiellem Befall zugesetzt. Hier kommen vor allem Ester der p-Hydroxybenzoesäure, Benzoesäure und deren Natriumsalze sowie Sorbinsäure und Kaliumsorbat zum Einsatz. Bei schwer zu konservierenden Präparaten können auch 2-Hydroxybiphenyl, Bromchlorophen, Dehydracetsäure oder Chlorhexidindigluconat eingesetzt werden. Ein weiterer Vorteil der erfindungsgemäßen Mittel besteht aber weiterhin darin, dass Chitosan selbst mikrobizid ist und so der Gehalt an zusätzlichen Konsverbierungsmitteln vergleichsweise gering ist.

### Beispiele

**Beispiel H1.** In einem 500-ml-Dreihalskolben mit Rührer und Rückflußkühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 0,5 g Allantoin in 88 ml Wasser und dann mit einer Zubereitung von 25 g Chitosan (Hydagen® DCMF, 1 Gew.-%ig in Glycolsäure, Henkel KGaA, Düsseldorf/FRG), 10 g Paraffinöl, 0,5 g Phenonip® (Konservierungmittelmischung enthaltend Phenoxyethanol und Parabene) und 0,5 g Polysorbat-20 (Tween® 20, ICI) in 64 g Wasser versetzt. Die erhaltene Matrix wurde filtriert, auf 60 °C erwärmt und in eine 0,5 Gew.-%ige Natriumalginatlösung getropft. Zum Erhalt von Mikrokapseln gleichen Durchmessers wurden die Zubereitungen anschließend gesiebt.

**Beispiel H2.** In einem 500-ml-Dreihalskolben mit Rührer und Rückflußkühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin in ad 100 g Wasser und dann mit einer Zubereitung von 25 g Chitosan (Hydagen® DCMF, 1 Gew.-%ig in Glycolsäure, Henkel KGaA, Düsseldorf/FRG), 2 g Pfefferminzöl und 0,5 g Phenonip® (in ad 100 g Wasser versetzt. Die erhaltene Matrix wurde filtriert, auf 50 °C temperiert und unter starken Rühren im 2,5fachen Volumen Paraffinöl, das zuvor auf 15 °C gekühlt worden war, dispergiert. Die Dispersion wurde anschließend mit einer wäßrigen Lösung enthaltend 1 Gew.-% Natriumlaurylsulfat und 0,5 Gew.-% Natriumalginat und dann mehrfach mit einer 0,5 Gew.-%igen wäßrigen Phenoniplösung gewaschen, wobei die Ölphase entfernt wurde. Nach dem Sieben wurde eine wäßrige Zubereitung erhalten, die 8 Gew.-% Mikrokapseln mit einem mittleren Durchmesser von 1 mm enthielt.

**Beispiel H3.** In einem 500-ml-Dreihalskolben mit Rührer und Rückflußkühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 0,2 g Azacycloheptandiphosphonat in 88 ml Wasser und dann mit einer Zubereitung von 2,5 g Natriumalginat in Form einer 10 Gew.-%igen wäßrigen Lösung, 1 g Pfefferminzöl, 0,5 g Phenonip® und 0,5 g Polysorbat-20 (Tween® 20, ICI) in 64 g Wasser versetzt. Die erhaltene Matrix wurde filtriert, auf 60 °C erwärmt und in eine 1 Gew.-%ige Lösung von Chitosanglycolat in Wasser getropft. Zum Erhalt von Mikrokapseln gleichen Durchmessers wurden die Zubereitungen anschließend gesiebt.

**Beispiel H4.** In einem 500-ml-Dreihalskolben mit Rührer und Rückflußkühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 2,5 g Natriumalginat in Form einer 10 Gew.-%igen wäßrigen Lösung, 0,3 Kochenillerot und 0,5 g Phenonip® (in ad 100 g Wasser versetzt. Die erhaltene Matrix wurde filtriert, auf 50 °C temperiert und unter starken Rühren im 2,5fachen Volumen Paraffinöl, das zuvor auf 15 °C gekühlt worden war, dispergiert. Die Dispersion wurde anschließend mit einer wäßrigen Lösung enthaltend 1 Gew.-% Natriumlaurylsulfat und 0,5 Gew.-% Chitosanglycolat und dann mehrfach mit einer 0,5 Gew.-%igen wäßrigen Phenoniplösung gewaschen, wobei die Ölphase entfernt wurde. Nach dem Sieben wurde eine wäßrige Zubereitung erhalten, die 8 Gew.-% Mikrokapseln mit einem mittleren Durchmesser von 1 mm enthielt.

In der nachfolgenden Tabelle 1 finden sich eine Reihe von Formulierungsbeispielen.

**Tabelle 1**

| **Formulierungsbeispiele** | | | | | | | | |
|---|---|---|---|---|---|---|---|---|
| **Zusammensetzung** | **1** | **2** | **3** | **4** | **5** | **6** | **7** | **8** |
| Mikrokapseln gemäß Bsp. 1 | - | - | - | - | - | 0,5 | - | - |
| Mikrokapseln gemäß Bsp. 2 | 1,0 | 1,0 | 1,0 | 1,1 | 1,0 | 0,1 | 3,5 | 0,15 |
| Mikrokapseln gemäß Bsp.3 | - | - | - | 1,0 | - | - | - | - |
| Mikrokapseln gemäß. Bsp.4 | - | - | | - | 0,1 | | 0,1 | 0,1 |
| Natriumfluorid | - | - | 0,2 | 0,2 | - | 0,2 | - | 0,1 |
| Natriummonofluorphosphat | 0,8 | 1,1 | - | - | - | - | - | - |
| Tetrakaliumdiphosphat | - | - | - | - | - | 4,5 | - | - |
| Tetranatriumdiphosphat | - | - | - | - | - | 1,5 | - | 1,4 |
| Strontiumchlorid-hexahydrat | - | - | - | - | 10,5 | - | - | - |
| Titandioxid | - | - | - | - | - | 1,0 | - | - |
| Carrageenan | - | - | - | - | - | 0,9 | - | - |
| Saccharin-Natrium | 0,1 | 0,1 | 0,1 | 0,2 | 0,4 | 0,4 | - | 0,1 |
| Natriumcyclamat | - | 0,1 | - | - | - | - | 0,2 | - |
| Abrasivkieselsäure | - | - | 11,0 | 15,0 | - | - | - | - |
| Fällungskieselsäure | - | - | - | - | 15,0 | 20,0 | - | - |
| Verdickungskieselsäure | - | - | 6,0 | 5,0 | - | - | - | - |
| Calciumcarbonat | 35,0 | - | - | - | - | - | - | - |
| Calciumhydrogenphosphat-dihydrat | - | 25,0 | | | | | | |
| Calciumhydrogenphosphat | - | 7,0 | - | - | - | - | - | - |
| Siliciumdioxid, hochdispers | 2,5 | 2,0 | - | - | 1,6 | - | - | - |
| Ethanol | - | - | - | - | - | - | 80,0 | 7,0 |
| Sorbit, 70 Gew.-%ig | 20,0 | 15,0 | 72,0 | 29,0 | 12,0 | 25,0 | - | - |
| Glycerin, 86 Gew.-%ig | - | 10 | | 12,5 | 18,0 | 15,0 | | 12,0 |
| PHB-Methylester | 0,1 | 0,1 | 0,1 | 0,1 | 0,1 | 0,1 | - | - |
| PHB-Propylester | 0,1 | - | - | - | 0,1 | - | - | - |
| Benzoesäure | - | 0,2 | | | | | | |
| Sorbinsäure | - | - | - | - | - | - | - | 0,2 |
| Bromchlorophen | - | - | - | 0,1 | - | - | - | - |
| Natriumlaurylsulfat | 1,5 | - | - | 1,0 | 1,3 | - | - | - |
| Natriumlauroylsarcosinat | - | 1,5 | - | - | - | 1,3 | - | - |
| Fettsäuretaurid | - | - | 1,5 | - | - | - | - | - |
| Hydroxyethylcellulose | - | - | - | - | 1,4 | - | - | - |
| Natriumcarboxymethylcellulose | 1,3 | 1,1 | 0,4 | 1,0 | - | - | - | - |
| PEG 1500 | - | - | 3,0 | - | - | 3,0 | - | - |
| Guar Gum | - | - | - | - | 0,6 | - | - | - |
| Wasser | ad 100 | | | | | | | |

| | | | | | | | | |
|---|---|---|---|---|---|---|---|---|
| (1,2) Anti-Karieszahncreme, (3) Gel-Zahncreme, (4) Zahncreme gegen Zahnstein, (5,6) Zahncreme für empfindliche Zähne, (7) Mundwasserkonzentrat, (8) Mundwasser | | | | | | | | |

## Patentansprüche

1. Mund- und Zahnpflegemittel, enthaltend mit Wirkstoffen und Gelbildnern ausgewählt aus der Gruppe der Agarosen, Pektine, Xanthane und Proteine beladene Chitosanmikrokapseln, die man erhält, indem man
(a1) aus Gelbildnern, Chitosanen und Wirkstoffen eine Matrix zubereitet und
(a2) die Matrix mit wässrigen Lösungen anionischer Polymere behandelt;
oder
(b1) aus Gelbildnern, Chitosanen und Wirkstoffen eine Matrix zubereitet,
(b2) die Matrix in einer Ölphase dispergiert,
(b3) die dispergierte Matrix mit wässrigen Lösungen anionischer Polymere behandelt und dabei die Ölphase entfernt;
oder
(c1) aus Gelbildnern, anionischen Polymeren und Wirkstoffen eine Matrix zubereitet und
(c2) die Matrix mit wässrigen Chitosanlösungen behandelt;
oder
(d1) aus Gelbildnern, anionischen Polymeren und Wirkstoffen eine Matrix zubereitet,
(d2) die Matrix in einer Ölphase dispergiert,
(d3) die dispergierte Matrix mit wässrigen Chitosanlösungen behandelt und dabei die Ölphase entfernt.

2. Zubereitungen nach Anspruch 1, **dadurch gekennzeichnet, dass** sie Wirkstoffe enthalten, die ausgewählt sind aus der Gruppe, die gebildet wird von Mitteln gegen die Erkrankung von Zähnen, Zahnhälsen und des Zahnfleischs, antiinflammatorischen Wirkstoffen, Bakteriziden sowie Aromakomponenten.

3. Zubereitungen nach den Ansprüchen 1 und/oder 2, **dadurch gekennzeichnet, dass** die Mikrokapseln Chitosane enthalten, die ein mittleres Molekulargewicht im Bereich von 10.000 bis 500.000 bzw. 800.000 bis 1.200.000 Dalton aufweisen.

4. Zubereitungen nach mindestens einem der Ansprüche 1 bis 3, **dadurch gekennzeichnet, dass** die Mikrokapseln als anionische Polymere Salze der Alginsäure oder anionische Chitosanderivate enthalten.

5. Verwendung von Chitosanmikrosphären nach Anspruch 1 zur Herstellung von Mund- und Zahnpflegemitteln.

## Claims

1. Oral and dental care preparations containing chitosan microcapsules charged with active principles and gel formers selected from the group of agaroses, pectins, xanthans and proteins, the chitosan microcapsules being obtained by
(a1) preparing a matrix from gel formers, chitosans and active substances and
(a2) treating the matrix with aqueous solutions of anionic polymers;
or
(b1) preparing a matrix from gel formers, chitosans and active substances,
(b2) dispersing the matrix in an oil phase,
(b3) treating the dispersed matrix with aqueous solutions of anionic polymers and removing the oil phase in the process,
or
(c1) preparing a matrix from gel formers, anionic polymers and active principles and
(c2) treating the matrix with aqueous chitosan solutions,
or
(d1) preparing a matrix from gel formers, anionic polymers and active principles,
(d2) dispersing the matrix in an oil phase,
(d3) treating the dispersed matrix with aqueous chitosan solutions and removing the oil phase in the process.

2. Preparations as claimed in claim 1, **characterized in that** they contain active principles selected from the group consisting of agents against disease of the teeth, teeth necks and gums, anti-inflammatory agents, bactericides and flavour components.

3. Preparations as claimed in claims 1 and/or 2, **characterized in that** the microcapsules contain chitosans which have an average molecular weight of 10,000 to 500,000 or 800,000 to 1,200,000 dalton.

4. Preparations as claimed in at least one of claims 1 to 3, **characterized in that** the microcapsules contain salts of alginic acid or anionic chitosan derivatives as the anionic polymers.

5. The use of the chitosan microcapsules according to claim 1 for the production of oral and dental care preparations.

## Revendications

1. Composition de soins bucco-dentaires, contenant des microcapsules de chitosane chargées en principes actifs et en gélifiants choisis dans le groupe des agaroses, des pectines, des xanthanes et des protéines, que l'on obtient
(a1) en préparant une matrice à partir de gélifiants, de chitosanes et de principes actifs et
(a2) en traitant la matrice avec des solutions aqueuses de polymères anioniques ;
ou
(b1) en préparant une matrice à partir de gélifiants, de chitosans et de principes actifs,
(b2) en dispersant la matrice dans une phase huileuse, et
(b3) en traitant la matrice dispersée avec des solutions aqueuses de polymères anioniques et en éliminant la phase huileuse ;
ou
(c1) en préparant une matrice à partir de gélifiants, de polymères anioniques et de principes actifs, et
(c2) en traitant la matrice avec des solutions aqueuses de chitosane ;
ou
(d1) en préparant une matrice à partir de gélifiants, de polymères anioniques et de principes actifs,
(d2) en dispersant la matrice dans une phase huileuse, et
(d3) en traitant la matrice dispersée avec des solutions aqueuses de chitosane et en éliminant la phase huileuse.
2°) Préparations selon la revendication 1,
**caractérisées en ce qu'**
elles contiennent des principes actifs choisis dans le groupe constitué de produits contre les pathologies des dents, des collets et des gencives, de principes actifs anti-inflammatoires, de bactéricides ainsi que de composants aromatiques.
3°) Préparations selon les revendications 1 et/ou 2,
**caractérisées en ce qu'**
elles contiennent des microcapsules de chitosanes qui ont un poids moléculaire moyen dans la plage de 10.000 à 500.000, ou de 800.000 à 1.200.000 Dalton.
4°) Préparations selon au moins l'une quelconque des revendications 1 à 3,
**caractérisées en ce que**
les microcapsules contiennent, comme polymères anioniques, des sels de l'acide alginique ou des dérivés anioniques de chitosane.
5°) Utilisation de microsphères de chitosane selon la revendication 1, pour fabriquer des produits de soins bucco-dentaires.
